Europäisches Patentamt

European Patent Office

Office européen des brevets

(11) Veröffentlichungsnummer: **0 250 998 B1**

# EUROPÄISCHE PATENTSCHRIFT

(45) Veröffentlichungstag der Patentschrift: **15.04.92**

(51) Int. Cl.⁵: **C12M  1/04**

(21) Anmeldenummer: **87108555.1**

(22) Anmeldetag: **13.06.87**

(54) **Vorrichtung und Verfahren zur Kultivierung von immobilisierten Mikroorganismen.**

(30) Priorität: **26.06.86 DE 3621328**

(43) Veröffentlichungstag der Anmeldung:
**07.01.88 Patentblatt  88/01**

(45) Bekanntmachung des Hinweises auf die
Patenterteilung:
**15.04.92 Patentblatt  92/16**

(84) Benannte Vertragsstaaten:
**AT BE CH DE ES FR GB IT LI NL SE**

(56) Entgegenhaltungen:
**EP-A- 0 112 095**
**EP-A- 0 154 334**
**US-A- 3 717 552**

**PATENT ABSTRACTS OF JAPAN, Band 8, Nr.
205 (C-243)[1642], 19. September 1984; & JP-
A-59 95 882 (KUBOTA TEKKO K.K.) 02-06-1984**

(73) Patentinhaber: **BAYER AG**

**W-5090 Leverkusen 1 Bayerwerk(DE)**

(72) Erfinder: **Kauling, Jörg, Dipl.-Ing.**
**Händelstrasse 53**
**W-5000 Köln(DE)**
Erfinder: **Henzler, Hans-Jürgen, Dr.**
**Eggenweg 30**
**W-5650 Solingen(DE)**
Erfinder: **Pascik, Imre, Dr.**
**Wilmerdorfer Strasse 6**
**W-4019 Monheim(DE)**
Erfinder: **Müller, Claus, Dipl.-Ing.**
**Zur Eiche 37**
**W-5067 Kürten(DE)**
Erfinder: **Baumgarten, Jörg, Dr.**
**Henselweg 13**
**W-5600 Wuppertal(DE)**
Erfinder: **Molitor, Alois**
**Mühlenweg 140**
**W-5090 Leverkusen(DE)**

## Beschreibung

Die Erfindung betrifft einen Wirbelbettreaktor zur Kultivierung von immobilisierten im Abwasser suspendierten Bio-Katalysatoren mit Einbauten zur Begasung und einem Rückhaltesystem für die immobilisierten Bio-Katalysatoren. Außerdem betrifft die Erfindung ein Verfahren zum Betrieb des Wirbelbettreaktors.

Bei der Abwasserreinigung und bei enzymatischen Reaktionen werden in zunehmendem Maße Mikroorganismen bzw. Enzyme an spezielle Träger gebunden, um eine Aktivitätserhöhung der Biokatalysatoren zu erreichen. Als Reaktoren kommen Festbett-und Wirbelbettreaktoren zum Einsatz. In Festbetten werden durch eine unregelmäßige Pakkung oder durch einen unregelmäßigen Bewuchs mit Organismen die Bio-Katalysatoren häufig nur teilweise genutzt. Außerdem kann durch Konzentrationsgradienten, die im Festbett vorliegen, der Prozeß ungünstig beeinflußt werden (z.B. durch pH-Wert-Änderungen). Für viele Prozesse wird daher besser ein Wirbelbettreaktor verwendet, bei dem eine ausreichende Vermischung der Flüssigphase erfolgt, so daß die Träger sich im suspendierten Zustand befinden oder zeitweilig in einen aufgewirbelten Zustand gebracht werden können.

Zum Aufwirbeln wird die Energie entweder über die Flüssigphase (durch Umpumpen) oder durch Begasen des Flussigkeits-Feststoff-Gemisches eingetragen. Bei aeroben Fermentationsprozessen bietet sich insbesondere das gasbetriebene Wirbelbett als Reaktortyp an. Eine Reduzierung des Energieeintrags zum Aufwirbeln der Bio-Katalysatoren ist durch Einbau von Leiteinrichtungen, wie z.B. Leitbleche oder Leitrohre im Reaktor möglich. Die im gasbetriebenen Wirbelbett häufig auftretenden fluktuierenden Bewegungen bewirken bei großen Anlagen starke Schwingungen der Einbauten, die schon oft zur Zerstörung der Leiteinrichtungen bzw. der Verankerungen geführt haben.

Bei kontinuierlicher Prozeßführung besteht ein weiteres Problem in der Rückhaltung der häufig kostenaufwendigen Bio-Katalysatoren im Reaktor. Die übliche Methode der Rückhaltung durch Filtration ist in vielen Fällen nicht praktikabel, da die Filter verstopfen oder/und nur geringe Filtratdurchsätze erreicht werden, die ihrerseits sehr groß Filterflächen erfordern und damit große Kosten verursachen.

Der Erfindung liegt die Aufgabe zugrunde, in einem Wirbelbettreaktor die Kultivierung von immobilisierten, suspendierten Bio-Katalysatoren durch eine verbesserte Begasung unter Berücksichtigung wirtschaftlicher Gesichtspunkte (niedrige Investitions-und Betriebskosten) zu verbessern.

Diese Aufgabe wird bei einem Wirbelbettreaktor mit Einbauten zur Begasung und einem integrierten Rückhaltesystem für die immobilisierten Bio-Katalysatoren erfindungsgemäß dadurch gelöst, daß die Einbauten zur Begasung aus mehreren am Boden des Wirbelbettreaktors angeordneten Segmenten mit Gasaustrittsöffnungen bestehen, die alternierend oder umlaufend mit Gas beaufschlagt werden.

Vorteilhaft ist als weitere Einbaueinheit zur Begasung eine zentrale, nach oben gerichtete Gasaustrittsöffnungen vorgesehen.

Um Verstopfungen zu vermeiden sind die Gasaustrittsöffnungen des Gasverteilers um einen Winkel von $\beta$ = 30° bis 90° von der Horizontalen zum Boden geneigt. Der Abstand der Gasaustrittsöffnungen voneinander beträgt 0,0 bis 1 m, der Öffnungswinkel $\alpha$ der Segmente $\alpha$ = 30° bis 180°, vorzugsweise 60° bis 120°. Die Anzahl der Segmente kann zwischen 2 und 12 und ihre radiale Ausdehnung zwischen r = D/10 und D/2, vorzugsweise zwischen D/5 und D/2 variieren (D = Reaktordurchmesser). Der Durchmesser der zentralen Begasungseinheit sollte 5 % bis 10 % des Reaktordurchmessers D betragen. Vorteilhaft können mehrere Einzelsegmente zu einer Begasungseinheit zusammengeschaltet werden.

Erfolgt die Begasung durch ein einziges Begasungssegment oder durch die zentrale Begasungseinheit, so bildet sich eine ausgeprägte Zirkulationsströmung aus, die bei deutlich niedrigeren Gasdurchsätzen (etwa nur 70 bis 80 % gegenüber der üblichen Flächenbegasung) zum Aufwirbeln der Feststoffe führt. Vergrößert man den Gasdurchsatz im Falle der Begasung mit einem Segment bis die Gasgeschwindigkeit so hoch ist, daß eine völlige Suspendierung erreicht wird, so ist überraschenderweise zu beobachten, daß der Feststoff zunächst gegenüber der Begasungsstelle, dann an den Stellen, die im rechten Winkel zum Begasungsort liegen und zuletzt erst in der Umgebung der Begasungsstelle aufgewirbelt wird.

Auf Grund dieses Sachverhaltes eröffnet sich eine bessere Möglichkeit zur Begasung, die darin besteht, daß die Segmente nacheinander in Umfangsrichtung mit Gas beaufschlagt werden (umlaufende Begasung). Dabei kann ebenfalls mit wesentlich geringeren Gasdurchsätzen (nur etwa 40 % der bei der Flächenbegasung notwendigen Gasmenge) gearbeitet werden. Darüber hinaus kann auch ein Wechsel der Begasung zwischen einzelnen oder allen Segmenten und der Mittenbegasung (alternierende Begasung) sowie die Kombination der umlaufenden und alternierenden Begasung in Betracht gezogen werden.

Bei der umlaufenden bzw. alternierenden Begasung sollte die Begasung zumindest über einen so langen Zeitraum erfolgen, bis sich ein stationärer Strömungszustand im Reaktor ausgebildet hat. Die Zeitintervalle zwischen dem Umschalten von einem zum anderen Begasungselement sollten aber klein gegenüber der mittleren Verweilzeit der Flüssigphase im Reaktor sein, damit Feststoffablagerungen nicht zu einer Ausbeuteminderung führen.

Die Rückhaltung der Bio-Katalysatoren erfolgt vorteilhaft durch einen außerhalb des Reaktors angekoppelten Umlaufabscheider. Die Katalysatorpartikel, die mit dem Flüssigkeitsstrom den Reaktor verlassen, werden im Umlaufabscheider durch Sedimentation zurückgehalten und strömen, bedingt durch die Umlaufströmung, in den Reaktor zurück. Die Umlaufströmung bildet sich aufgrund des unterschiedlichen Gasgehaltes im Reaktor und im Umlaufabscheider von selbst aus. Zur Unterstützung der Strömung werden in den Umlaufabscheider zweckmäßig Umlenkelemente und/oder Siebplatten eingebaut. Eine weitere Möglichkeit, eine Umlaufströmung zu erzeugen besteht darin, daß in der Nähe des Suspensionsauslaufstutzens zusätzlich eine geringe Menge Gas zugeführt wird.

Der Umlaufabscheider und seine Zu- und Abfuhrleitungen müssen so groß dimensioniert werden, daß sich kein Feststoff ablagern kann, der zum Verstopfen führt. Der Abstand h zwischen dem Ein- und dem Auslaufstutzen sollte mindestens 0,5 m betragen, damit Störungen der Sedimentation durch die Einlaufströmung weitgehend vermieden werden.

Im folgenden wird ein Ausführungsbeispiel der Erfindung anhand von Zeichnungen näher erläutert. Es zeigen:

Fig. 1 schematisch eine Seitenansicht des Reaktors mit angekoppeltem Umlaufabscheider,

Fig. 2 einen Querschnitt A-A durch den Reaktor nach Fig. 1 in Höhe des Gasverteilers und

Fig. 3a-6a verschiedene Einbauten im Umlaufabscheider.

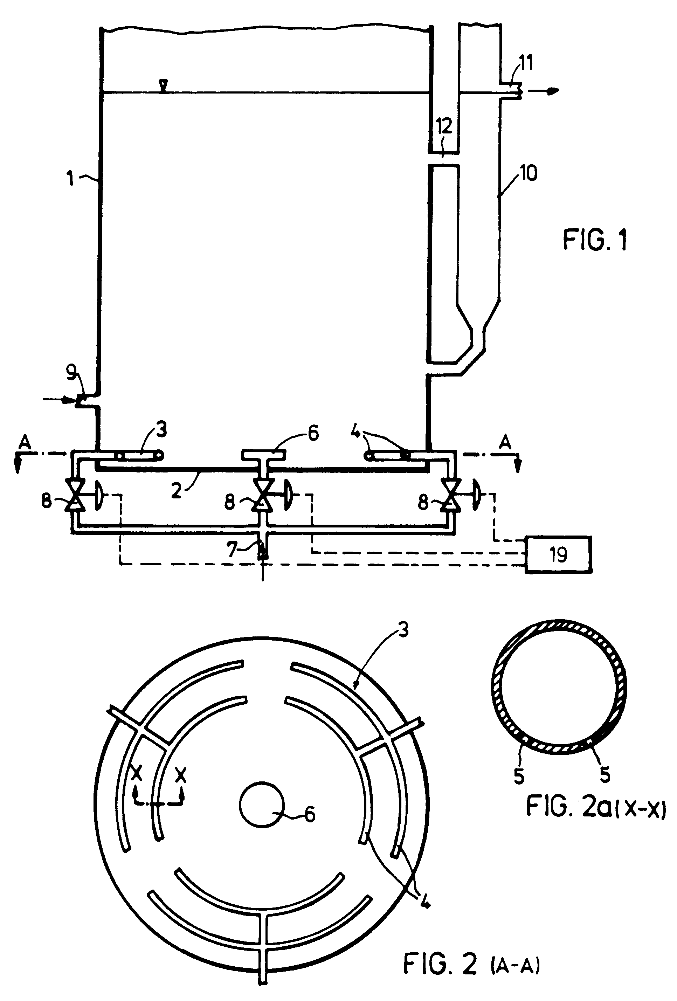

Der Reaktor gemäß Fig. 1 besteht aus einem zylindrischen Behälter 1 von 5 m Durchmesser und 5 m Höhe mit ebenem Boden 2. Dicht über dem Behälterboden 2 ist ein aus drei Segmenten 3 mit einem Winkel $\alpha$ = 100° aufgebauter Gasverteiler angeordnet. Der Gasverteiler besteht aus zwei ringförmigen Rohrleitungen 4, die im Abstand von 25 cm von der Behälterwand und 50 cm voneinander montiert sind. Die Rohrleitungen 4 sind im Abstand von 25 cm mit nach unten geneigten Bohrungen 5 von 4 mm Durchmesser versehen. In der Mitte befindet sich außerdem eine zentrale Begasungseinheit 6.

Jedes Begasungssegment 3 bzw. Einheit 6 kann über ein Regelventil 8 angesteuert werden und ist mit der Hauptgasleitung 7 verbunden. Durch eine zentrale Regeleinheit 19 werden die Ventile 8 so angesteuert, daß die Einbauten 3 bzw. 6 nacheinander mit Gas beaufschlagt werden, wodurch eine umlaufende oder alternierende Begasung erzeugt wird.

Im Reaktor befinden sich 30 Vol.-% Polyurethan-Schaum-Flocken, die mit Bakterien zur Abwasserreinigung bewachsen sind. Die mittlere Größe der Partikel beträgt dabei 4 mm und die Dichte im wäßrigen Zustand 1040 kg/m³. Durch eine Gasmenge von nur 100 m³/h, was einer spezifischen Leistung von etwa 14 Watt/m³ und einer Gasleerrohrgeschwindigkeit von 5 m/h entspricht, wird dieser Bio-Katalysator in bewegtem Zustand ehalten, so daß ein Zusammenwachsen von Partikeln und damit eine Reduzierung der Katalysatorkapazität vermieden wird. Damit läßt sich im Dauerbetrieb eine kontinuierliche Reinigung von problematischen Industrieabwässern durchführen.

Der Abwasserstrom, der über den Stutzen 9 in den Reaktor einströmt, verläßt durch den an den Bioreaktor angekoppelten Umlaufabscheider 10 über den Auslaufstutzen 11 die Anlage. Nachdem in der Anfahrphase der biologischen Reinigung die durch den Herstellungsprozeß des Bio-Katalysators bedingten Feinanteile ausgetragen wurden, wird das Trägermaterial im Abscheider durch Sedimentation zurückgehalten. Für einen Abwasserdurchsatz von 10 m³/h besitzt der Abscheider nur einen Durchmesser von 500 mm. Die Länge des Umlaufabscheiders zwischen Ein- (12) und Auslauf (13) beträgt etwa 2 m.

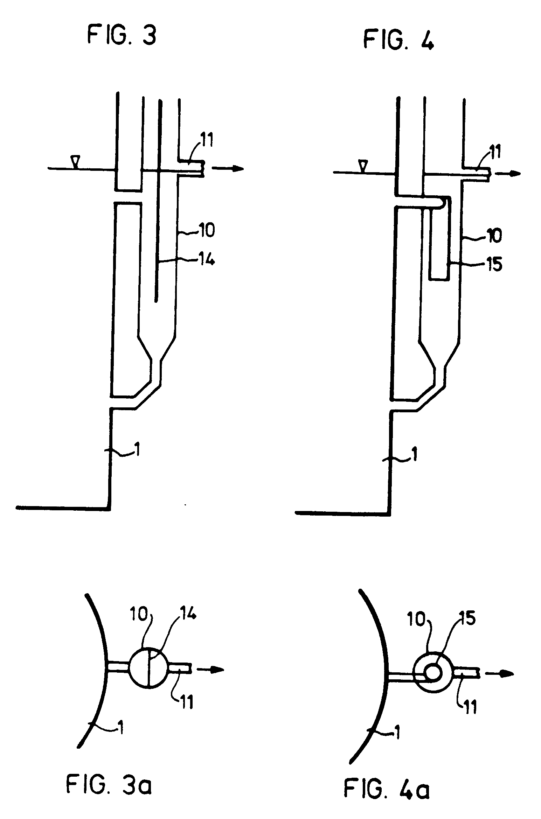

Für den Fall, daß aufgrund der geringen Höhe des Wirbelbettes auch die Bauhöhe des Umlaufabscheiders klein gehalten werden muß, wird man auf die Einbauten nach Fig. 3a bis 6a zurückgreifen. Gemäß Fig. 3a und 4a wird der Abstand zwischen der Stelle der Strömungsumlenkung im Abscheider und im Auslauf durch den Einbau eines Schwertes 14 bzw. einer Beruhigungsstrecke 15 vergrößert und damit die Abscheideleistung verbessert.

Gemäß den Ausführungen 5a bis 6a wird der Umlaufabscheider 10 zusätzlich mit Siebplatten 16 ausgerüstet. Derartige Siebplatten 16 eignen sich insbesondere zur Rückhaltung von Feinanteilen des Bio-Katalysators. Dieses Problem tritt jedoch nur dann auf, wenn sich die Partikel, bedingt durch das Herstellungsverfahren oder durch den Abrieb im Reaktor, sehr stark in ihrer Größe unterscheiden.

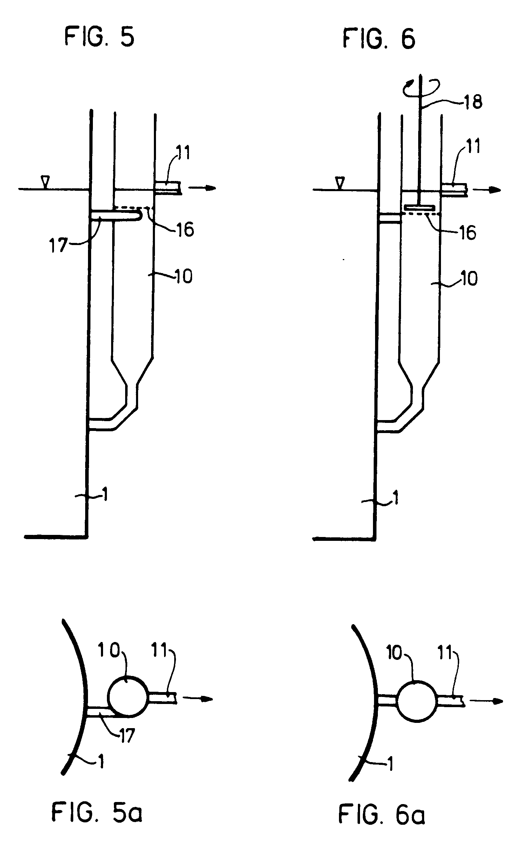

Um zu vermeiden, daß sich die Siebplatten 16 im Betrieb zusetzen und damit verstopfen, werden sie durch einen tangential einmündenden Flüssigkeitsstrahl 17 oder einen oberhalb angeordneten Rührer 18 angeströmt. Durch die tangentiale Anströmung nach Fig. 5a oder durch den Rührer 18 nach Fig. 6a wird eine Drallströmung erzeugt, die eine gleichmäßige Spülung der Siebplatten 16 bewirkt.

**Patentansprüche**

1. Wirbelbett-Reaktor zur Kultivierung von immobilisierten, im Abwasser suspendierten Bio-Katalysatoren mit Einbauten zur Begasung und einem Rückhaltesystem für die immobilisierten Bio-Katalysatoren, dadurch gekennzeichnet, daß die Einbauten zur Begasung aus mehreren am Boden des Wirbelbett-Reaktors angeordneten Segmenten (3) mit Gasaustrittsöffnungen (5) bestehen, die alternierend oder umlaufend mit Gas beaufschlagt werden.

2. Wirbelbett-Reaktor nach Anspruch 1, dadurch gekennzeichnet, daß als weitere Einbaueinheit zur Begasung eine zentrale, nach oben gerichtete Gasaustrittsöffnung (6) vorgesehen ist.

3. Wirbelbettreaktor nach Anspruch 1, dadurch gekennzeichnet, daß das Rückhaltesystem für die immobilisierten Biokatalysatoren als Umlaufabscheider (10) ausgebildet ist.

4. Wirbelbett-Reaktor nach Anspruch 1 bis 3, dadurch gekennzeichnet, daß der Umlaufabscheider (10) außerhalb des Reaktors angeordnet ist.

5. Wirbelbett-Reaktor nach Anspruch 1 bis 4, dadurch gekennzeichnet, daß im Umlaufabscheider Umlenkelemente (14), (15) und/oder Siebplatten (16) eingebaut sind.

6. Wirbelbett-Reaktor nach Anspruch 1 bis 5, dadurch gekennzeichnet, daß in der Nähe des Auslaufstutzens (12) zur Unterstützung der Umlaufströmung ein Gaszuführungsstutzen angebracht ist.

7. Verfahren zum Betrieb des Wirbelbett-Reaktors nach Anspruch 1 bis 6, dadurch gekennzeichnet, daß durch sequentielles Öffnen der Gaszuführungsöffnungen (5) an den segmentförmigen Einbauten (3) eine umlaufende Begasung im Reaktor erfolgt.

8. Verfahren zum Betrieb des Wirbelbett-Reaktors nach Anspruch 1 bis 6, dadurch gekennzeichnet, daß von der Begasung durch die zentrale Gasaustrittsöffnung (6) auf eine Begasung durch die segmentförmigen Einbauten (3) oder vice versa umgeschaltet wird.

9. Verfahren nach Anspruch 7 bis 8, dadurch gekennzeichnet, daß durch tangentiale Einleitung (17) der Suspension im Umlaufabscheider (10) eine Drallströmung erzeugt wird.

**Claims**

1. Fluidized-bed reactor for cultivating immobilized biocatalysts suspended in waste water, having gassing installations and a retention system for the immobilized biocatalysts, characterized in that the gassing installations comprise a plurality of segments (3) with gas outlet openings (5) which are disposed at the bottom of the fluidized-bed reactor and are acted upon by gas alternately or in rotation.

2. Fluidized-bed reactor according to claim 1, characterized in that a central, upwardly directed gas outlet opening (6) is provided as a further gassing installation unit.

3. Fluidized-bed reactor according to claim 1, characterized in that the retention system for the immobilized biocatalysts takes the form of a rotary separator (10).

4. Fluidized-bed reactor according to claims 1 to 3, characterized in that the rotary separator (10) is disposed outside of the reactor.

5. Fluidized-bed reactor according to claims 1 to 4, characterized in that baffle elements (14), (15) and/or perforated plates (16) are installed in the rotary separator.

6. Fluidized-bed reactor according to claims 1 to 5, characterized in that a gas inlet port is provided in the vicinity of the discharge port (12) to boost the circulation flow.

7. Process for operating the fluidized-bed reactor according to claims 1 to 6, characterized in that a gas circulation is effected in the reactor by sequentially opening the gas inlet openings (5) at the segment-like installations (3).

8. Process for operating the fluidized-bed reactor according to claims 1 to 6, characterized in that there is a switchover from gassing through the central gas outlet opening (6) to gassing through the segment-like installations (3) or vice versa.

9. Process according to claims 7 to 8, characterized in that a swirling flow is generated in the rotary separator (10) as a result of the tangential introduction (17) of the suspension.

**Revendications**

1. Réacteur à lit tourbillonnaire pour la culture de biocatalyseurs immobilisés en suspension dans des eaux usées, comportant des chicanes pour l'introduction de gaz et un système de retenue pour les biocatalyseurs immobilisés, caractérisé en ce que les chicanes pour l'introduction de gaz sont constituées de plusieurs segments (3), disposés à la base du réacteur à lit tourbillonnaire, présentant des orifices (5) de sortie de gaz, gui sont alimentés en gaz en alternance ou en circuit.

2. Réacteur à lit tourbillonnaire suivant la revendication 1, caractérisé en ce qu'il est prévu comme autre unité incorporée pour l'introduction de gaz une sortie centrale de gaz (6) dirigée vers le haut.

3. Réacteur à lit tourbillonnaire suivant la revendication 1, caractérisé en ce que le système de retenue pour les biocatalyseurs immobilisés est réalisé comme séparateur (10) à circulation.

4. Réacteur à lit tourbillonnaire suivant les revendications 1 à 3, caractérisé en ce que le séparateur à circulation (10) est disposé en dehors du réacteur.

5. Réacteur à lit tourbillonnaire suivant les revendications 1 à 4, caractérisé en ce que des éléments déflecteurs (14), (15) et/ou des plaques perforées (16) sont incorporés dans le séparateur à circulation.

6. Réacteur à lit tourbillonnaire suivant les revendications 1 à 5, caractérisé en ce qu'un manchon d'arrivée de gaz est disposé à proximité du manchon de sortie (12) pour entretenir le courant de circulation.

7. Procédé pour faire fonctionner le réacteur à lit tourbillonnaire suivant les revendications 1 à 6, caractérisé en ce qu'une circulation de gaz dans le réacteur est créée par l'ouverture en séquence des orifices (5) d'amenée de gaz prévues sur les chicanes (3) en forme de segments.

8. Procédé pour faire fonctionner le réacteur à lit tourbillonnaire suivant les revendications 1 à 6, caractérisé par le passage de l'arrivée de gaz par l'orifice central (6) de sortie de gaz à une arrivée de gaz par les chicanes (3) en forme de segments ou vice versa.

9. Procédé suivant les revendications 7 et 8, caractérisé par le fait qu'un écoulement avec effet de spin est produit par l'entrée tangentielle (17) de la suspension dans le séparateur à circulation (10).

FIG. 1

FIG. 2 (A-A)

FIG. 2a (x-x)

## FIG. 3

## FIG. 4

## FIG. 3a

## FIG. 4a

FIG. 5

FIG. 6

FIG. 5a

FIG. 6a